# EUROPÄISCHE PATENTSCHRIFT

(11) **EP 2 948 537 B1**
(45) Veröffentlichungstag und Bekanntmachung des Hinweises auf die Patenterteilung: **22.11.2017**
(21) Anmeldenummer: 14700878.3
(22) Anmeldetag: 16.01.2014
(51) Int. Cl.: C12M 1/00

(54) **FERMENTERBESCHICKUNGSVERFAHREN, BIOGASANLAGE UND UMRÜSTUNGSVERFAHREN**
FERMENTER SUPPLY METHOD, BIOGAS PLANT, AND CONVERSION METHOD
PROCÉDÉ D'ALIMENTATION D'UN FERMENTATEUR, INSTALLATION À BIOGAZ ET PROCÉDÉ DE TRANSFORMATION

(30) Priorität: 25.01.2013 CH 300132013
(43) Veröffentlichungstag der Anmeldung: 02.12.2015
(73) Patentinhaber: Hitachi Zosen Inova AG, 8005 Zürich (CH)
(72) Erfinder: OERTIG, Michael, CH-8500 Frauenfeld (CH); LEISNER, Rene, 78467 Konstanz (DE)
(74) Vertreter: Schaad, Balass, Menzl & Partner AG
(86) Internationale Anmeldenummer: PCT/EP2014/050817
(87) Internationale Veröffentlichungsnummer: WO 2014/114557

(56) Entgegenhaltungen:
- EP-A1- 1 930 404
- EP-A2- 0 621 336
- WO-A1-2010/085893
- DE-A1- 4 307 334
- DE-A1-102009 009 985
- GB-A- 720 018
- GB-A- 2 029 392

## Beschreibung

### Technisches Gebiet

Die vorliegende Erfindung beschreibt ein Fermenterbeschickungsverfahren für eine Biogasanlage, eine Biogasanlage, umfassend eine Mehrzahl von im Pfropfstrombetrieb geführten Fermenterkammern, sowie ein Umrüstungsverfahren einer Biogasanlage mit mindestens einer im Pfropfstrombetrieb geführten Fermenterkammer.

### Stand der Technik

Es sind unterschiedliche Ausführungsformen von Fermentern für die anaerobe Fermentation von Biomasse zur Verwendung in Biogasanlagen bekannt.

So sind pfropfstrombetriebene liegende Fermenter der Anmelderin bekannt, die zur anaeroben Fermentation biogener Abfälle geeignet sind und gemäss dem Verfahren des europäischen Patentes EP621336 arbeiten. Der Fermenter ist ein langgestreckter, liegender Tank mit einem an einem Ende vorgesehenen Einlass und einem am gegenüber liegenden Ende vorhandenen Auslass. Die biogenen Abfälle werden einlassseitig zerkleinert eingegeben und mit fermentiertem Gut und Presswasser aus der Aufbereitung geimpft. Hierdurch wird das zu fermentierende Gut mit Methanbakterien angereichert. Im Fermenter werden nun unter kontrollierter Durchmischung die biogenen Abfälle unter Bildung von Biogas abgebaut und anschliessend nach dem Verlassen durch den Auslass einer aeroben Verrottung zugeführt.

Die weltweite Nachfrage nach Anlagen der eingangs genannten Art mit immer grösseren Kapazitäten führt dazu, dass auch immer grössere Fermenter gebaut werden. Um dies zu ermöglichen, müssen die Fermenter in Form von Fermentertanks aus Stahl oder aus Beton vor Ort erstellt werden. Zur Erhöhung der Kapazität werden heute liegende Fermentertanks mit einer Gesamtlänge von 50 Metern und mehr, bei Durchmessern von über 10 Metern realisiert. Ein darin betriebenes Rührwerk muss nicht nur die biogenen Abfälle durchmischen um eine gewisse Homogenität zu erreichen, sondern gleichzeitig muss sichergestellt werden, dass schwere Festgüter, wie insbesondere Sand und Steine nicht am Boden des Fermentertanks sedimentieren und folglich nicht mehr ausgetragen werden. Obwohl der Fermenter im Pfropfstrom betrieben wird, vermag die Durchströmung die absinkenden Schwerstoffe nicht auszutragen, da die Pfropfstrombewegung nur eine geringe Strömungsgeschwindigkeit aufweist. Die Durchsatzzeit der biogenen Abfälle durch den Fermenter vom Einlass zum Auslass beträgt mehrere Tage.

Das Rührwerk trägt folglich neben der Durchmischung ebenfalls dazu bei, diese Schwerstoffe vom Boden wieder nach oben zu befördern, um danach bei der nachfolgenden Sinkbewegung im Pfropfstrom Richtung Fermenterauslass transportiert zu werden. Entsprechend besteht das Rührwerk aus einer den Fermenter durchsetzenden Welle mit einer Vielzahl von Rührarmen, die an ihrem von der Welle abgelegenen Ende mit entsprechenden Schaufeln versehen sind.

Die technischen Schwierigkeiten betreffend den Transport des Gärsubstrates durch den Fermenter, sowie die Steuerung der Fermentationsprozesse durch Messung und Optimierung von Parametern wie Temperatur, Wasseranteil des Gärsubstrates und pH-Wert sind soweit gelöst. Auch die Realisierung von Fermentationsverfahren, welche eine Schonung der Fermenterbauteile erlaubt, ist beispielsweise aus der EP1841853 bekannt.

Um den Gesamtdurchsatz des Gärsubstrats durch eine Biogasanlage zu steigern, kann die Verweilzeit des Gärsubstrates im Fermenter reduziert werden, was in der EP1930404 offenbart ist. Aus der EP1930404 geht ein Verfahren zur Fermentation hervor, wobei teilweise vergorenes Impfsubstrat aus einem Auslass des Fermenters in den selben Fermenter zurückgeführt wird und als Impfmittel benutzt wird. Eine derartige Nutzung teilweise vergorenen Impfsubstrates aus dem Fermenter selbst, wird damit zur Eigenimpfung der frischen Biomasse im Bereich des Eingangs des Fermenters verwendet. Dadurch kann der Durchsatz durch den einen Fermenter gesteigert werden und die Verweilzeit auf wenige Tage reduziert werden.

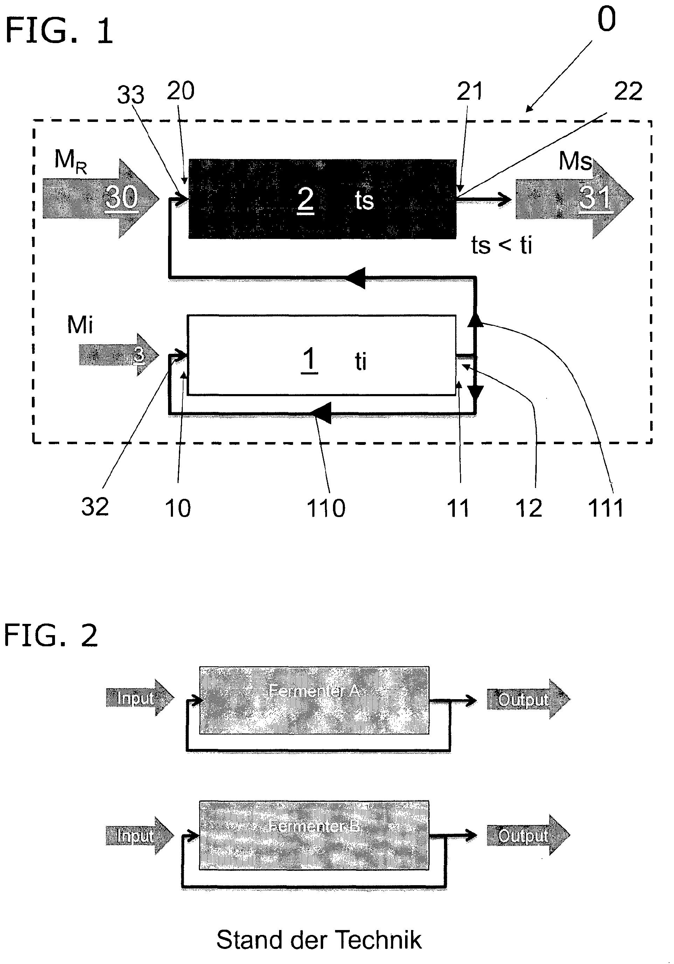

Der Gesamtdurchsatz der Biomasse einer Biogasanlage kann entsprechend durch eine Mehrzahl von gemäss EP1930404 beschriebenen Fermentern gesteigert werden, welche parallel zueinander betrieben werden. Der derart maximal erreichbare Gesamtdurchsatz beträgt entsprechend Anzahl der Fermenter mal Durchsatz jedes Fermenters. Dies ist beispielhaft in der Figur 2 dargestellt. In diesem Fall ist der Austrag aus beiden Fermentern verdoppelt, während die Verweilzeit der Biomasse in den Fermentern gleich ist und jeweils ein Anteil des Austrages zur Eigenimpfung des jeweiligen Fermenters genutzt wird.

Auf die klassische Eigenimpfung verzichtet die GB720018. Aus der GB720018 geht ein anaerobes Fermentierungsverfahren von Biomasse hervor, welches mehrere Fermenterkammern benutzt, durch welche Gärsubstrat in einer vorgegebenen Reihenfolge durchgeführt wird. Es wird kein Hinweis gegeben, dass durch das Verfahren gemäss GB720018 der Durchsatz von Biomasse durch die Biogasanlage gesteigert werden kann oder sollte. Es wird die bei der Fermentation im Schnellfermenter auftretende Flüssigkeit (digester liquor) vom Schnellfermenter in den getrennt angeordneten Impffermenter geführt, wodurch Bakterien im Impffermenter wiederverwendet werden können. Es ist nicht offenbart und auch für den Fachmann nicht zu erkennen, ob dieses Vorgehen zu einem gesteigerten Durchsatz führt. Durch diese Massnahme kann eine Wiederverwendung der Bakterien erreicht werden, was zu einer Optimierung der Arbeitsabläufe und zu einer gewissen Kosteneinsparung führt. Die Idee der Eigenimpfung aus der EP1930404 führt von der Idee der Rückführung der im Schnellfermenter auftretenden Flüssigkeit vom Schnellfermenter in den getrennt angeordneten Impffermenter aus der GB720018 weg, sodass keine Kombination beider Verfahren nahegelegt wird und der Fachmann von einer Kombination weggeführt wird.

### Darstellung der Erfindung

Die vorliegende Erfindung hat sich die Aufgabe gestellt, ein Verfahren zu schaffen, mittels welchem der Gesamtdurchsatz des Substrats durch eine Mehrzahl von Fermenterkammern in einer Biogasanlage gesteigert wird, wobei die Anzahl der Fermenterkammern nicht verändert wird. Durch minimalen apparativen Aufwand kann ein Gesamtdurchsatz durch die Biogasanlage erreicht werden, der grösser als der n-fache Durchsatz durch jede einzelne Fermenterkammer ist.

Obwohl eine Mehrzahl von Fermenterkammern genutzt wird, sind die notwendigen Investitionen in die Anlagentechnik gerechnet auf den erreichbaren Gesamtdurchsatz kleiner als bei der simplen parallelen Anordnung von n nicht gekoppelten Fermenterkammern.

Diese Aufgabe wird durch eine spezielle Beschickung und Kopplung der Mehrzahl von Fermenterkammern gelöst. Dadurch, dass Eigenimpfsubstrat aus einer im Pfropfstrombetrieb geführten Impffermenterkammer über einen Rückführpfad zurück in die Impffermenterkammer geleitet und zusätzlich über einen Schnellimpfpfad in mindestens eine weitere, von der Impffermenterkammer unabhängige im Pfropfstrombetrieb geführte Schnellfermenterkammer geleitet und dort mit weiterem frischem Rohgärsubstrat vermengt wird. In der Schnellfermenterkammer kann dadurch eine wesentlich kürzere Verweilzeit erreicht werden und der Gesamtdurchsatz des Rohgärsubstrates der Biogasanlage verglichen mit einer Biogasanlage mit gleicher Anzahl parallel betriebener Fermenterkammern vergrössert werden.

### Kurze Beschreibung der Zeichnungen

Ein bevorzugtes Ausführungsbeispiel des Erfindungsgegenstandes wird nachstehend im Zusammenhang mit den anliegenden Zeichnungen beschrieben.
- Figur 1: zeigt eine schematische Ansicht einer Biogasanlage mit einer Impffermenterkammer und einer Schnellfermenterkammer, welche miteinander gekoppelt sind und gemäss erfindungsgemässem Verfahren mit Substrat beschickt werden.
- Figur 2: zeigt eine schematische Ansicht eines ersten und eines zweiten Fermenters mit Eigenimpfung, welche parallel betrieben werden, gemäss Stand der Technik.

### Beschreibung

In dieser Anmeldung werden verschiedene Fermenterkammern, in Form von abgeschlossenen Räumen offenbart, welche räumlich voneinander getrennt sein können, oder aneinander angrenzend angeordnet sein können. Die jeweiligen Innenräume der Fermenterkammern sind voneinander getrennt und durch unterschiedliche Leitungen oder Pfade miteinander gekoppelt.

In Figur 1 ist eine Biogasanlage 0 gezeigt, welche hier beispielhaft eine Impffermenterkammer 1 und eine, mit der Impffermenterkammer 1 gekoppelte Schnellfermenterkammer 2 aufweist. In der hier dargestellten Biogasanlage 0 weisen beide Fermenterkammern identische Innenraumgrössen und damit gleiche Nutzvolumen auf, welche mit Gärsubstraten beladen oder beschickt werden können. Wahlweise kann die Biogasanlage 0 mehrere Impffermenterkammern 1 und/oder mehrere Schnellfermenterkammern 2 aufweisen, welche wie im Folgenden beschrieben gekoppelt und beschickbar sind.
Das Verfahren ist auch auf Fermenterkammern mit jeweils unterschiedlichem Nutzvolumen anwendbar, wird aber hier beispielhaft an identischen Nutzvolumen beschrieben.

Von einer Eintragsseite 10 wird die Impffermenterkammer 1 mit Rohgärsubstrat 3 beschickt, welches im Pfropfstrombetrieb in Richtung einer Austragsseite 11 der Impffermenterkammer 1 geführt wird. Das Rohgärsubstrat 3 wird in der Impffermenterkammer 1 innerhalb einer Verweilzeit ti von wenigen Tagen fermentiert, bis vergorenes Impffermentersubstrat resultiert.

Aus einem Ausgang 12 der Austragsseite 11 wird Eigenimpfsubstrat 32 als Teil des vergorenen Impffermentersubstrats über einen Rückführpfad 110 zurück zur Eintragsseite 10 der Impffermenterkammer 1 und wieder in den Innenraum der Impffermenterkammer 1 geführt. Das Eigenimpfsubstrat 32 wird dabei mit frischem Rohgärsubstrat 3 gemischt, wodurch ein Substratgemisch erzeugt wird. Dieses Substratgemisch wird anschliessend beim Durchgang durch die Impffermenterkammer 1 im pfropfstrombetrieb fermentiert. Die Beschickung der Impffermenterkammer 1 wird damit mit einer Selbstimpfung von frischem Rohgärsubstrat mit vergorenem Eigenimpfsubstrat 32 aus der Impffermenterkammer 1 selbst durchgeführt. Insgesamt wird damit die Impffermenterkammer 1 mit einer Menge Mi von Rohgärsubstrat pro Zeiteinheit beschickt, welche einen Teil der Gesamtdurchsatzmenge Ms der Biogasanlage 0 pro Zeiteinheit bildet.

Ein weiterer Teil des vergorenen Impffermentersubstrats aus der Impffermenterkammer 1 wird als Schnellfermenterimpfsubstrat 33 bezeichnet, aus dem Ausgang 12 über einen Schnellimpfpfad 111 zu einer Eintragsseite 20 der Schnellfermenterkammer 2 geführt. Dort wird das Schnellfermenterimpfsubstrat 33 mit einer Menge MR frischen Schnellfermenterrohsubstrats 30 in die Schnellfermenterkammer 2 geleitet, sodass eine Fremdimpfung des frischen Schnellfermenterrohsubstrats 30 in der Schnellfermenterkammer 2 durchgeführt wird. Ebenfalls im Pfopfstrombetrieb wird das Gemisch aus Schnellfermenterimpfsubstrat 33 und frischem Schnellfermenterrohsubstrat 30 durch die Schnellfermenterkammer 2 bis zu einer Austragsseite 21 geführt und dabei fermentiert. Aus einem Ausgang 22 wird dann nach einer Verweilzeit ts in der Schnellfermenterkammer 2 vergorenes Ausgangssubstrat 31 abgeführt. Insgesamt wird eine Gesamtmenge Ms vergorenen Substrates durch die Biogasanlage 0 durchgeführt und fermentiert. Diese Gesamtmenge Ms pro Zeiteinheit setzt sich aus der Summe der Menge des Rohgärsubstrates durch die Impffermenterkammer Mi und der Menge Schnellfermenterrohsubstrates MR zusammen, wobei Ms kleiner als die Summe aus Mi + MR ist, da etwa 10 % der Masse in Biogas umgesetzt werden.

Beide Fermenterkammern 1, 2 werden gemäss vorgestelltem Beschickungsverfahren mit unterschiedlichen Mengen Mi, MR frischen Rohgärsubstrates beschickt. Dies ist dadurch möglich, dass die Fermentation in der Schnellfermenterkammer 2 wesentlich schneller abläuft, als in der Impffermenterkammer 1, da die Schnellfermenterkammer 2 sich nicht selbst impft. Da sich die Schnellfermenterkammer 2 nicht selbst impfen muss, kann die Verweilzeit ts in der Schnellfermenterkammer 2 wesentlich reduziert werden. Demnach ist die Verweilzeit ti des Substrates in der Impffermenterkammer 1 grösser als die Verweilzeit des Substrates in der Schnellfermenterkammer 2.

Dadurch verdoppelt sich die Menge MR Schnellfermenterrohsubstrat pro Zeiteinheit und damit der Schnellfermenterdurchsatz nahezu und der Gesamtdurchsatz der Biogasanlage 0 kann deutlich im Vergleich zum Betrieb zweier baugleicher Fermenterkammern in parallelem Betrieb gesteigert werden.

Der Aufbau einer derartigen Biogasanlage 0 benötigt nur jeweils einen Rückführpfad 110 pro Impffermenterkammer 1, wobei aus der Austragsseite 21 der Schnellfermenterkammer 2 vergorenes Ausgangssubstrat 31 direkt zur weiteren Verwendung entnommen werden kann.

Es konnte gezeigt werden, dass bei Verwendung einer Impffermenterkammer 1 mit einem Durchsatz Mi von 20000 Mg/a und einer baugleichen Schnellfermenterkammer 2 mit einem Durchsatz MR von 35000 Mg/a ein Gesamtdurchsatz von 55000 Mg/a erreicht werden konnte. Bei Betrieb der Fermenterkammern auf die klassische Weise mittels Selbstimpfung ist nur ein Gesamtdurchsatz von 40000 Mg/a erreichbar, da jede Fermenterkammer mit Selbstimpfung einen identischen Durchsatz von 20000 Mg/a aufweist.

Der Gasertrag in der Biogasanlage 0 gemäss Figur 1 fällt allerdings etwas geringer aus, als bei Parallelbetrieb zweier Fermenterkammern. Der Grund dafür ist die vergleichsweise kürzere Verweilzeit ts in der Schnellfermenterkammer 2.

Zum Vergleich des erfindungsgemässen Beschickungsverfahrens mit dem Stand der Technik sind in der Tabelle 1 zwei Biogasanlagen gegenübergestellt. In einer Ausführungsform gemäss Stand der Technik sind drei baugleiche Fermenterkammern nebeneinander betrieben worden, wodurch insgesamt ein Gesamtjahresdurchsatz aller drei Fermenterkammern von 60000 Megagramm pro Jahr erreicht wird. Jede der unabhängig von den anderen Fermenterkammern betriebene Fermenterkammer trägt ein Drittel des Gesamtjahresdurchsatzes bei.

In einer Biogasanlage mit einer Impffermenterkammer und einer Schnellfermenterkammer, bei Betrieb mit dem erfindungsgemässen Beschickungsverfahren, können bereits 55000 Megagramm pro Jahr Gesamtjahresdurchsatz erreicht werden, wobei eine Fermenterkammer weniger eingesetzt wird.

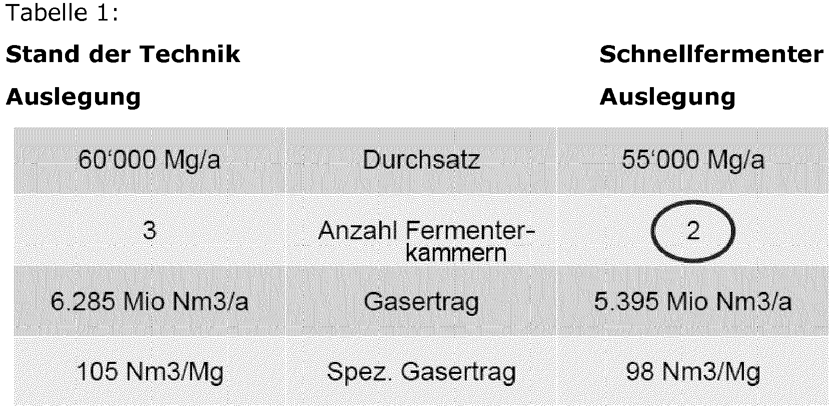

In einer Versuchsanlage wurden zwei Fermenterkammern 1, 2 mit identischem Nutzvolumen gekoppelt und mit Substraten beschickt. In Tabelle 2 werden die eingestellten Parameter der Beschickung und die technischen Details der Fermenterkammern 1, 2 aufgeführt.

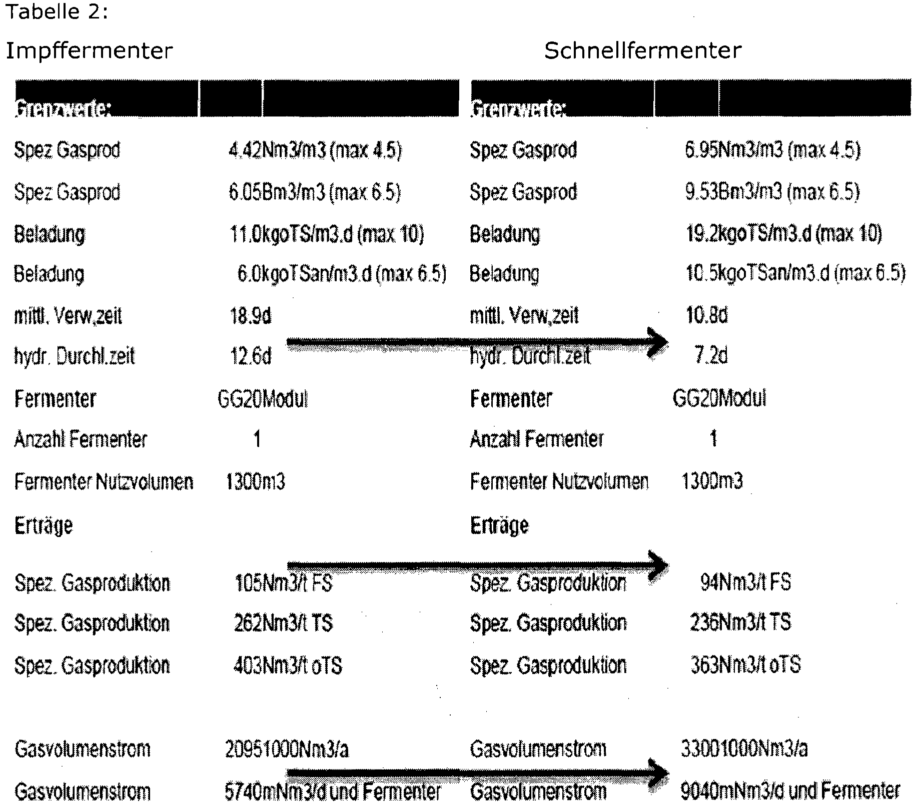

Neben der dargestellten räumlich getrennten Anordnung der Impffermenterkammer 1 und der Schnellfermenterkammer 2, können diese auch direkt benachbart mit sehr kurz ausgeführten Pfaden 110, 111 oder direkt aneinander grenzend angeordnet sein, wodurch eine kompakte Biogasanlage 0 ausbildbar ist.

Bevorzugt wird eine Impffermenterkammer 1 gekoppelt mit zwei oder drei Schnellfermenterkammern 2 vorgesehen. Dadurch kann ausreichend Schnellfermenterimpfsubstrat 33 bereitgestellt werden, um den Betrieb der Mehrzahl von Schnellfermenterkammern 2 zu optimieren.

Es ist möglich, bestehende Biogasanlagen mit mindestens einer Fermenterkammer umzurüsten, um diese mit dem hier beschriebenen Fermenterbeschickungsverfahren betreiben zu können. Dazu muss mindestens eine Impffermenterkammer 1 mit einem Rückführpfad 110 versehen und über einen Schnellimpfpfad 111 mit mindestens einer Schnellfermenterkammer 2 wirkverbunden gekoppelt werden. Um den Durchsatz von Rohsubstrat durch eine Biogasanlage zu steigern, ist ein solches Umrüstungsverfahren sinnvoll. Da oftmals in bekannten Biogasanlagen eine Mehrzahl von getrennten und parallel betriebenen Fermenterkammern vorhanden ist, besteht die Umrüstung aus der Kopplung von Schnellfermenterkammer 2 und Impffermenterkammer 1.

### Bezugszeichenliste

0 Biogasanlage
1 Impffermenterkammer
   10 Eintragsseite
   11 Austragseite
      110 Rückführpfad
      111 Schnellimpfpfad
   12 Ausgang
2 Schnellfermenterkammer
   20 Eintragsseite
   21 Austragsseite
   22 Kammerausgang
3 Rohgärsubstrat
   30 Schnellfermenterrohsubstrat
   31 vergorenes Ausgangssubstrat
   32 Eigenimpfsubstrat
   33 Schnellfermenterimpfsubstrat
ti Verweilzeit in Impffermenterkammer
ts Verweilzeit in Schnellfermenterkammer
Mi Menge durch Impffermenterkammer pro Zeiteinheit /Impfdurchsatz
MR Menge Schnellfermenterrohsubstrat pro Zeiteinheit / Schnellfermenterdurchsatz
Ms Gesamtmenge durch Biogasanlage pro Zeiteinheit/Gesamtdurchsatz

## Patentansprüche

1. Fermenterbeschickungsverfahren für eine Biogasanlage (0), **gekennzeichnet durch**
die Schritte:
Beschickung mindestens einer im Pfropfstrombetrieb geführten Impffermenterkammer (1) mit Rohgärsubstrat (3) einer Menge (Mi) pro Zeiteinheit und Fermentierung für eine Verweilzeit (ti),
Rückführung eines Teils des in der Impffermenterkammer (1) vergorenen Substrates in Form eines Eigenimpfsubstrates (32) über einen Rückführpfad (110) direkt zurück in die Impffermenterkammer (1) unter Mischung mit weiterem Rohgärsubstrat (3)
und
Beschickung mindestens einer, mit der Impffermenterkammer (1) gekoppelten, im Pfropfstrombetrieb geführten Schnellfermenterkammer (2) mit einem weiteren Teil des in der Impffermenterkammer (1) vergorenen Substrates in Form eines Schnellfermenterimpfsubstrates (33) und Mischung mit Schnellfermenterrohsubstrat (30) einer Menge (MR) pro Zeiteinheit, wobei eine Entnahme vergorenen Ausgangssubstrates (31) aus der Schnellfermenterkammer (2) jeweils nach einer Verweilzeit (ts) des in die Schnellfermenterkammer (2) eingefüllten Substratgemisches erfolgt.

2. Fermenterbeschickungsverfahren gemäss Anspruch 1, wobei die Verweilzeit (ts) in der Schnellfermenterkammer (2) kleiner als die Verweilzeit (ti) in der Impffermenterkammer (1) ist.

3. Fermenterbeschickungsverfahren gemäss Anspruch 1, wobei die Menge (Mi) des Rohgärsubstrates (3) mit der die Impffermenterkammer (1) pro Zeiteinheit beschickt wird, kleiner ist als die Menge (MR) des Schnellfermenterrohsubstrates pro Zeiteinheit mit der die Schnellfermenterkammer (2) beschickt wird.

4. Fermenterbeschickungsverfahren gemäss Anspruch 1, wobei jeweils ein Teil des in der Impffermenterkammer (1) vergorenen Substrates in die Impffermenterkammer (1) zurückgeführt wird und der andere Teil in die Schnellfermenterkammer (2) eingeführt wird.

5. Fermenterbeschickungsverfahren gemäss Anspruch 1, wobei die mindestens eine Impffermenterkammer (1) mit mehr als einer Schnellfermenterkammer (2) gekoppelt ist und die Fermenterkammern (1, 2) beschickt werden.

6. Biogasanlage (0), umfassend eine Mehrzahl von Fermenterkammern,
**dadurch gekennzeichnet, dass**
mindestens eine erste im Pfropfenstrombetrieb geführte Impffermenterkammer (1)
mit einem Rückführpfad (110) zur Eigenimpfung
und
mindestens einem Schnellimpfpfad (111) zur Beschickung mindestens einer im Pfropfenstrombetrieb geführten Schnellfermenterkammer (2) mit Schnellfermenterimpfsubstrat (33), als Teil des in der Impffermenterkammer (1) vergorenen Substrates, vorgesehen ist, wobei die Verweilzeit (ts) des Substrates in der, an die mindestens eine Impffermenterkammer (1) gekoppelten mindestens einen Schnellfermenterkammer (2), kleiner ist, als die Verweilzeit (ti) des Substrates in der mindestens einen Impffermenterkammer (1).

7. Biogasanlage (0) gemäss Anspruch 6, wobei die Biogasanlage (0) mit dem Verfahren eines der Ansprüche 1 bis 5 betrieben wird.

8. Umrüstungsverfahren einer Biogasanlage mit einer Fermenterkammer, wobei
die Fermenterkammer als eine im Pfropfenstrombetrieb geführte Impffermenterkammer (1) mit einem Rückführpfad (110) zur Eigenimpfung mit Eigenimpfsubstrat (32) ausgestattet wird und eine im Pfropfenstrombetrieb geführte Schnellfermenterkammer (2) installiert wird, welche mit einem Schnellimpfpfad (111) an den Ausgang (12) der Impffermenterkammer (1) gekoppelt wird, sodass Schnellfermenterimpfsubstrat (33) in der Schnellfermenterkammer (2) mit Schnellfermenterrohsubstrat (30) mischbar und fermentierbar ist.

9. Umrüstungsverfahren einer Biogasanlage mit einer Mehrzahl von getrennten und parallel betriebenen Fermenterkammern, wobei
mindestens eine im Pfropfenstrombetrieb geführte Impffermenterkammer (1) mit einem Rückführpfad (110) versehen und über einen Schnellimpfpfad (111) mit mindestens einer im Pfropfenstrombetrieb geführte Schnellfermenterkammer (2) wirkverbunden gekoppelt wird.

## Claims

1. Fermenter supply method for a biogas plant (0), **characterized by** the steps of:
supplying at least one inoculum fermenter chamber (1) operated in the plug-flow mode with raw fermentation substrate (3) of an amount (Mi) per unit of time and fermenting for a dwell time (ti),
returning part of the substrate fermented in the inoculum fermenter chamber (1) in the form of a self-inoculating substrate (32) directly to the inoculum fermenter chamber (1) by means of a return path (110), said part being mixed with further raw fermentation substrate (3), and
supplying at least one fast fermenter chamber (2) operated in the plug-flow mode and coupled to the inoculum fermenter chamber (1) with an additional part of the substrate fermented in the inoculum fermenter chamber (1) in the form of a fast fermenter inoculation substrate (33) and mixing said additional part with fast fermenter raw substrate (30) of an amount (MR) per unit of time, wherein fermented output substrate (31) is removed from the fast fermenter chamber (2) each time after a dwell time (ts) of the substrate mixture introduced into the fast fermenter chamber (2).

2. Fermenter supply method according to claim 1, wherein the dwell time (ts) in the fast fermenter chamber (2) is shorter than the dwell time (ti) in the inoculum fermenter chamber (1).

3. Fermenter supply method according to claim 1, wherein the amount (Mi) of the raw fermentation substrate (3), with which the inoculum fermenter chamber (1) is supplied per unit of time, is smaller than the amount (MR) of the fast fermenter raw substrate per unit of time, with which the fast fermenter chamber (2) is supplied.

4. Fermenter supply method according to claim 1, wherein at any time one part of the substrate fermented in the inoculum fermenter chamber (1) is returned to the inoculum fermenter chamber (1) and the other part is supplied to the fast fermenter chamber (2).

5. Fermenter supply method according to claim 1, wherein the at least one inoculum fermenter chamber (1) is coupled to more than one fast fermenter chambers (2) and the fermenter chambers (1, 2) are supplied.

6. Biogas plant (0) comprising a plurality of fermenter chambers, **characterized in that**
at least a first inoculum fermenter chamber (1) operated in the plug-flow mode having a return path (110) for self-inoculation and at least one fast inoculation path (111) for supplying at least one fast fermenter chamber (2) operated in the plug-flow mode with fast fermenter inoculation substrate (33) being part of the substrate fermented in the inoculum fermenter chamber (1) is provided, wherein the dwell time (ts) of the substrate in the at least one fast fermenter chamber (2) coupled to the at least one inoculum fermenter chamber (1) is shorter than the dwell time (ti) of the substrate in the at least one inoculum fermenter chamber (1).

7. Biogas plant (0) according to claim 6, wherein the biogas plant (0) is operated according to the method of one of claims 1 to 5.

8. Conversion method for a biogas plant having a fermenter chamber, wherein
the fermenter chamber is equipped as an inoculum fermenter chamber (1) operated in the plug-flow mode with a return path (110) for self-inoculation by means of self-inoculating substrate (32) and a fast fermenter chamber (2) operated in the plug-flow mode is installed, which is coupled to an outlet (12) of the inoculum fermenter chamber (1) by means of a fast inoculation path (111), such that it is possible to mix and ferment fast fermenter inoculation substrate (33) in the fast fermenter chamber (2) with fast fermenter raw substrate (30).

9. Conversion method for a biogas plant having plurality of separate fermenter chambers operated in parallel, wherein at least one inoculum fermenter chamber (1) operated in the plug-flow mode is equipped with a return path (110) and is operatively coupled by means of a fast inoculation path (111) to at least one fast fermenter chamber (2) operated in the plug-flow mode.

## Revendications

1. Méthode d'alimentation d'un fermenteur pour une usine de biogaz (0), **caractérisée par** les étapes:
alimenter au moins une chambre de fermentation pour l'inoculation (1) fonctionnant à effet piston avec du substrat de fermentation brut (3) dans une quantité (Mi) par unité de temps et fermenter pour une durée de séjour (ti),
retourner une partie du substrat fermenté dans la chambre de fermentation pour l'inoculation (1) sous la forme d'un substrat auto-inoculant (32) directement à la chambre de fermentation pour l'inoculation (1) au moyen d'un chemin de retour (110) avec mélange avec du substrat de fermentation brut (3) supplémentaire, et
alimenter au moins une chambre de fermentation rapide (2) fonctionnant à effet piston couplée à la chambre de fermentation pour l'inoculation (1) avec une partie supplémentaire du substrat fermenté dans la chambre de fermentation pour l'inoculation (1) sous la forme d'un substrat d'inoculation pour fermenteur rapide (33) et mélanger avec du substrat brut de fermenteur rapide (30) dans une quantité (MR) par unité de temps, du substrat fermenté sortant (31) étant extrait de la chambre de fermentation rapide (2) chaque fois après une durée de séjour (ts) du mélange de substrat introduit dans la chambre de fermentation rapide (2).

2. Méthode d'alimentation d'un fermenteur selon la revendication 1, la durée de séjour (ts) dans la chambre de fermentation rapide (2) étant plus courte que la durée de séjour (ti) dans la chambre de fermentation pour l'inoculation (1).

3. Méthode d'alimentation d'un fermenteur selon la revendication 1, la quantité (Mi) du substrat de fermentation brut (3) avec laquelle la chambre de fermentation pour l'inoculation (1) est alimentée par unité de temps étant plus petite que la quantité (MR) du substrat brut de fermenteur rapide avec laquelle la chambre de fermentation rapide (2) est alimentée par unité de temps.

4. Méthode d'alimentation d'un fermenteur selon la revendication 1, une partie du substrat fermenté dans la chambre de fermentation pour l'inoculation (1) étant retourné chaque fois dans la chambre de fermentation pour l'inoculation (1) et l'autre partie étant introduite dans la chambre de fermentation rapide (2).

5. Méthode d'alimentation d'un fermenteur selon la revendication 1, la au moins une chambre de fermentation pour l'inoculation (1) étant couplée à plus d'une chambre de fermentation rapide (2) et les chambres de fermentation (1, 2) étant alimentées.

6. Usine de biogaz (0) comprenant une multitude de chambres de fermentation, **caractérisée en ce qu'**est prévue au moins une première chambre de fermentation pour l'inoculation (1) fonctionnant à effet piston avec un chemin de retour (110) pour l'auto-inoculation et au moins un chemin d'inoculation rapide (111) pour alimenter au moins une chambre de fermentation rapide (2) fonctionnant à effet piston avec du substrat d'inoculation pour fermenteur rapide (33), comme partie du substrat fermenté dans la chambre de fermentation pour l'inoculation (1), la durée de séjour (ts) du substrat dans la au moins une chambre de fermentation rapide (2) couplée à la au moins une chambre de fermentation pour l'inoculation (1) étant plus courte que la durée de séjour (ti) du substrat dans la au moins une chambre de fermentation pour l'inoculation (1).

7. Usine de biogaz (0) selon la revendication 6, l'usine de biogaz (0) fonctionnant avec la méthode de l'une des revendications 1 à 5.

8. Méthode de conversion pour une usine de biogaz ayant une chambre de fermentation ,
la chambre de fermentation étant équipée en tant que chambre de fermentation pour l'inoculation (1) fonctionnant à effet piston avec un chemin de retour (110) pour l'auto-inoculation au moyen d'un substrat auto-inoculant (32) et une chambre de fermentation rapide (2) fonctionnant à effet piston étant installée, laquelle est reliée à une sortie (12) de la chambre de fermentation pour l'inoculation (1) au moyen d'un chemin d'inoculation rapide (111), de telle sorte que du substrat d'inoculation pour fermenteur rapide (33) dans la chambre de fermentation rapide (2) peut être mélangé et fermenté avec du substrat brut de fermenteur rapide (30).

9. Méthode de conversion pour une usine de biogaz ayant une multitude de chambre de fermentation séparées fonctionnant en parallèle, au moins une chambre de fermentation pour l'inoculation (1) fonctionnant à effet piston étant équipée avec un chemin de retour (110) et étant couplée fonctionnellement au moyen d'un chemin d'inoculation rapide (111) avec au moins une chambre de fermentation rapide (2) fonctionnant à effet piston.
